# EUROPEAN PATENT APPLICATION

(11) **EP 1 736 149 A2**
(43) Date of publication of application: **27.12.2006**
(21) Application number: 06253223.9
(22) Date of filing: 22.06.2006
(51) Int. Cl.: A61K 31/122, A61K 31/23, A61K 31/231, A61P 3/00, A61P 3/06, A61P 9/00, A61P 9/10

(54) **Astaxanthin-containing agent for lowering neutral fat concentration in blood**

(30) Priority: 23.06.2005 JP 2005183016; 17.10.2005 JP 2005301155
(71) Applicant: YAMAHA HATSUDOKI KABUSHIKI KAISHA, Iwata-shi, Shizuoka 438-8501 (JP)
(72) Inventor: Murakami, Nagisa, Yamaha Hatsudoki KK, Iwata-shi, Shizuoka 438-8501 (JP); Okada, Yumika, Yamaha Hatsudoki KK, Iwata-shi, Shizuoka 438-8501 (JP)
(74) Representative: Schlich, George William

(57) **Abstract**

An agent for lowering neutral fat concentration in blood and a therapeutic agent for a disease to which neutral fat in blood is related, the agent containing astaxanthin and/or esters thereof . The agent for lowering neutral fat concentration in blood according to the present invention is useful for treating various diseases having an apparent relation to neutral fat in blood, such as arteriosclerosis, a cardiovascular disease, a cerebrovascular disorder, hyperlipemia, or a metabolic disorder.

## Description

### 1. Field of the Invention

The present invention relates to an agent for lowering neutral fat concentration in blood. More specifically, the present invention relates to an agent for lowering neutral fat concentration in blood and a therapeutic agent for a disease to which neutral fat in blood is related, the agents containing astaxanthin and/or an ester thereof.

### 2. Description of the Related Art

Generally, a condition in which the amounts of neutral fat and cholesterol in blood are much higher than standard (normal) values is referred to as hyperlipemia. Hyperlipemia is thought to be caused by excess intake of animal food products, lack of exercise, and other factors. Excess neutral fat in blood not only obstructs the blood flow, suppresses metabolism, and promotes thrombus formation, but also is a factor in metabolic syndrome, and therefore excess neutral fat is also an important risk factor associated with various life-style related diseases. Moreover, excess neutral fat in blood decreases HDL-cholesterol and increases LDL-cholesterol, and the excess neutral fat itself also accumulates on blood vessel walls, resulting in arteriosclerosis which is a cause of hypertension, cerebral thrombosis, and myocardial infarction.

Maintaining the amount of neutral fat in blood at a standard (normal) value is a safeguard against the above-mentioned diseases, and thus has attracted public interest. Once hyperlipemia develops, moderate exercise, reducing the amount of fat intake, ingesting more fruit and vegetables, and so on, are recommended for treatment.

Methods for treating pathological conditions such as hyperlipemia, in which the balance of the amounts of fats in blood is disturbed, include the ingestion of a polyunsaturated fatty acid such as linoleic acid and pharmacotherapy with clofibrate, nicotinic acid, or other drugs. For example, it is reported that a krill extract containing a large amount of ω-3 fatty acid decreases cholesterol *in vivo* (WO 02/102394). However, a problem with the first mentioned treatment is that it requires the ingestion of polyunsaturated fatty acid continuously for a long term, and excess intake of the polyunsaturated fatty acid is in itself problematic. As to the second mentioned treatment, clofibrate has side effects such as rhabdomyolysis; and nicotinic acid also has side effects such as systemic flushing and gastropathy. Thus, various studies have been conducted to find a drug that lowers fat concentration in blood, offers a high degree of safety, and can be used continuously for a long term. For example, the following material has been used for those purposes: components derived from naturally-occurring products, such as betaine contained in large amount in beet (Sugar beet) (Japanese Laid-Open Patent Publication No. 2003-261445); an analogue of curcumin contained in turmeric (Japanese Laid-Open Patent Publication No. 2003-2827); seed powder of the *Quercus crispula* and *Quercus serrata* trees (Japanese Laid-Open Patent Publication No. 11-299451); a low-polarity component of a water and/or polar organic solvent extract from a plant belonging to the genus *Beta* of the family chenopodiaceae (Japanese Laid-Open Patent Publication No. 7-69911).

### SUMMARY OF THE INVENTION

It is an object of the present invention to provide a novel agent for lowering neutral fat concentration in blood, with a high degree of safety. It is a further object of the present invention to provide a therapeutic agent for a disease having an apparent relation to neutral fat in blood.

The present invention provides an agent for lowering neutral fat concentration in blood, the agent comprising astaxanthin and/or an ester thereof.

The present invention also provides a therapeutic agent for a disease having a relation to neutral fat in blood, the agent comprising astaxanthin and/or an ester thereof.

In an embodiment, the disease is arteriosclerosis, a cardiovascular disease, a cerebrovascular disorder, hyperlipemia, or a metabolic disorder.

The present invention further provides an agent for controlling neutral fat concentration in blood, the agent comprising astaxanthin and/or an ester thereof.

The present invention also relates to use of astaxanthin and/or an ester thereof for lowering and/or controlling neutral fat concentration in blood. The use may be in vitro. The use may be cosmetic. Further provided is use of astaxanthin and/or an ester thereof in the manufacture of a medicament for treating a disease having a relation to neutral fat in blood. The disease may be treatable by lowering and/or controlling neutral fat concentration in blood.

Moreover, the present invention provides a method for lowering neutral fat concentration in blood, comprising administering an effective neutral fat lowering amount of astaxanthin and/or an ester thereof to an individual suffering from a disease having a relation to neutral fat in blood.

In addition, the present invention provides a method for preventing or treating a disease having a relation to neutral fat in blood, comprising administering a prophylactically or therapeutically effective amount of astaxanthin and/or an ester thereof to an individual suffering from a disease having a relation to neutral fat in blood.

The present invention also provides a method for controlling neutral fat concentration in blood, comprising administering an appropriate amount of astaxanthin and/or an ester thereof to an individual suffering from a disease having a relation to neutral fat in blood.

The agent of the present invention can also be used as a cosmetic agent for various conditions having an apparent relation to neutral fat in blood. Thus, the methods of the present invention may also relate to cosmetic methods, and the agents to cosmetic agents.

According to the present invention, a novel agent for lowering neutral fat concentration in blood and an agent for controlling neutral fat concentration in blood are provided. This agent for lowering neutral fat concentration in blood can be used as a therapeutic agent for various diseases having an apparent relation to neutral fat in blood, such as arteriosclerosis, cardiovascular diseases, cerebrovascular disorders, hyperlipemia, and metabolic disorders. The agent for lowering neutral fat concentration in blood of the present invention has very low toxicity and thus offers a high degree of safety.

### BRIEF DESCRIPTION OF THE DRAWINGS

Fig. 1 is a graph showing the change in the concentration of neutral fat in blood for different subjects before and after ingestion of astaxanthin capsules.
Fig. 2 is a graph showing the average values of the concentrations of neutral fat in blood of the subjects before and after the ingestion of the astaxanthin capsules.

### DESCRIPTION OF THE PREFERRED EMBODIMENTS

Astaxanthin or an ester thereof used in the present invention is a carotenoid represented by the following formula: 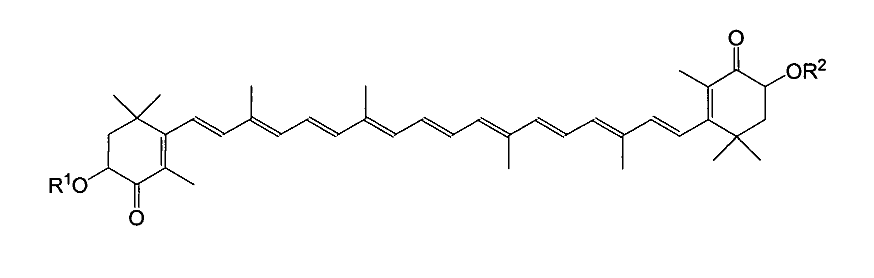 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue in the ester of astaxanthin include, but are not limited to, saturated fatty acids such as palmitic acid and stearic acid or unsaturated fatty acids such as oleic acid, linoleic acid, α-linolenic acid, γ-linolenic acid, bishomo-γ-linolenic acid, arachidonic acid, eicosapentaenoic acid, and docosahexaenoic acid. The astaxanthin ester of the present invention can be any mono- or diester, homogeneous or non-homogeneous. Astaxanthin has a structure in which an additional oxo group and an additional hydroxy group are present at each end of a β-carotene molecule, so that unlike for β-carotene, the stability of the molecule is low. On the other hand, an ester form (e.g., as obtained in an extract from krill) in which the hydroxy groups at both ends are esterified with an unsaturated fatty acid is more stable.

Astaxanthin or an ester thereof used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin and/or an ester thereof. In the present invention, any extract containing astaxanthin and/or esters thereof produced by any method can be used. Generally, extracts from those naturally-occurring products can be used, and the extracts may be crude or purified if necessary. In the present invention, a crude extract or a crushed powder of naturally-occurring products, or a purified product or a chemically synthesized product, if necessary, that contains such astaxanthin and/or esters thereof can be used either alone or in combination. In view of the chemical stability, an ester form of astaxanthin is preferably used.

The agent for lowering neutral fat concentration in blood of the present invention also can be used as an agent for controlling the concentration of neutral fat in blood. Since the agent can control the concentration of neutral fat in blood, it may be useful for treating or preventing diseases or symptoms exhibiting a relationship to neutral fat in blood. Examples of such diseases or symptoms include hyperlipemia, arteriosclerosis, hypertension, myocardial infarction, cerebrovascular disorders, cerebral infarction, angina pectoris, pancreatitis, diabetes, fatty liver, and metabolic disorders.

The therapeutic agent of the present invention for a disease having a relation to neutral fat in blood contains astaxanthin and/or esters thereof as an active component, just as is the case with the agent for lowering neutral fat concentration in blood according to the present invention described above. In particular, the therapeutic agent is useful for treating arteriosclerosis, cardiovascular diseases, cerebrovascular disorders, hyperlipemia, and metabolic disorders.

The route of administration of the agent for lowering neutral fat concentration in blood or the therapeutic agent of the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances commonly used in the field of pharmaceutical manufacturing technology, such as excipients, binders, antiseptics, antioxidants, disintegrators, lubricants, and flavoring agents, can be used as necessary.

The dose of the agent for lowering neutral fat concentration in blood or the therapeutic agent of the present invention varies depending on the purpose of administration, the individual to be administered to (sex, age, body weight, etc.), and the severity and nature of the disease, and can be determined by a person skilled in the art. Usually, the dose for an adult in terms of free or unesterified form of astaxanthin may be 0.1 mg to 2 g, preferably 4 mg to 500 mg per day in the case of oral administration, while it may be 0.01 mg to 1 g, preferably 0.1 mg to 500 mg per day in the case of parenteral administration.

The agent for lowering neutral fat concentration in blood according to the present invention can be used not only as pharmaceuticals as described above, but also as the category of products regulated as "quasi-drugs", cosmetics, food products, nutritional supplements, foods and drinks, and other similar products. When used as quasi-drugs or cosmetics, the neutral fat-depressing agent may be used in conjunction with various auxiliary substances commonly used in the field of quasi-drugs or cosmetics, or other technologies, if necessary. Alternatively, when used as food products, nutritional supplements, or foods and drinks, the neutral fat lowering agent may be used in conjunction with additives commonly used for food products, for example, sweeteners, spices, seasonings, antiseptics, preservatives, germicides, and antioxidants, if necessary. The neutral fat lowering agent may be used in a desired form such as a solution, suspension, syrup, granule, cream, paste, or jelly, or may be shaped, if necessary. The ratio of the neutral fat lowering agent contained in these products is not particularly limited, and can be selected appropriately according to the intended purpose, the mode of usage, and the amount of usage.

### Examples

### Preparation Example 1: Preparation of Astaxanthin

Astaxanthin was prepared in the following manner. *Haematococcus pluvialis* K0084 strain was cultivated at 25°C under irradiation with light while bubbling a gas containing 3% CO₂ into the medium and under nutrient stress condition (i.e. nitrogen source deprivation), and then was encysted. The encysted cells were disrupted by means commonly used by those skilled in the art, and a lipophilic fraction containing astaxanthin was extracted with ethanol. The extract was concentrated under reduced pressure, and the ethanol was evaporated to give an extract containing astaxanthin in an amount of 9.9% expressed in terms weight of the free form.

### Preparation Example 2: Preparation of Astaxanthin Monoester

An astaxanthin monoester was prepared in the following manner. *Haematococcus pluvialis* K0084 strain was cultivated at 25°C under irradiation with light while bubbling a gas containing 3% CO₂ into the medium and under nutrient stress condition (i.e. nitrogen source deprivation), and then was encysted. The encysted cells were disrupted by means commonly used by those skilled in the art, and a lipophilic fraction was extracted with ethanol. The extract contained lipids such as triglyceride in addition to astaxanthins. The extract was subjected to column chromatography using a synthetic resin adsorbent to give a purified product containing astaxanthin monoesters. This purified product was analyzed by HPLC, and it was confirmed that this purified product contained an astaxanthin monoester having a molecular weight of 858 as the main component, did not contain the free form of astaxanthin, the diester form of astaxanthin, and triglyceride, and that it contained a small amount of diglyceride.

### Preparation Example 3: Preparation of Astaxanthin Capsule

Soft capsules containing the components shown in Table 1 below per capsule were prepared using the astaxanthin obtained in Preparation Example 1.

**Table 1**

| Component | Weight | Note |
|---|---|---|
| Haematococcus extract | 40 mg | Astaxanthin 9.9 wt% |
| Glycerin fatty acid ester (Nikko Chemicals Co.. Ltd.) | 20 mg | as emulsifier |
| Safflower oil (The Nisshin OilliO Group. Ltd.) | 190 mg | |
| Total | 250 mg | |

The obtained soft capsules contained astaxanthin in an amount of 4 mg per capsule expressed in terms of weight of the free form.

### Example 1: Effect of Astaxanthin on Concentration of Neutral Fat in Blood

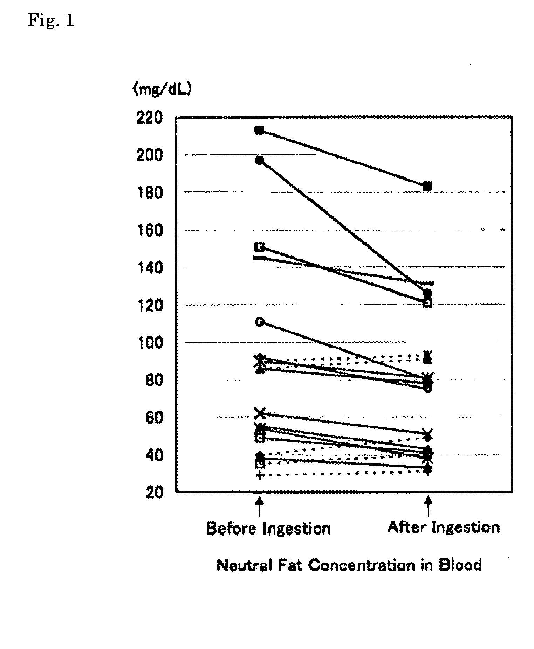

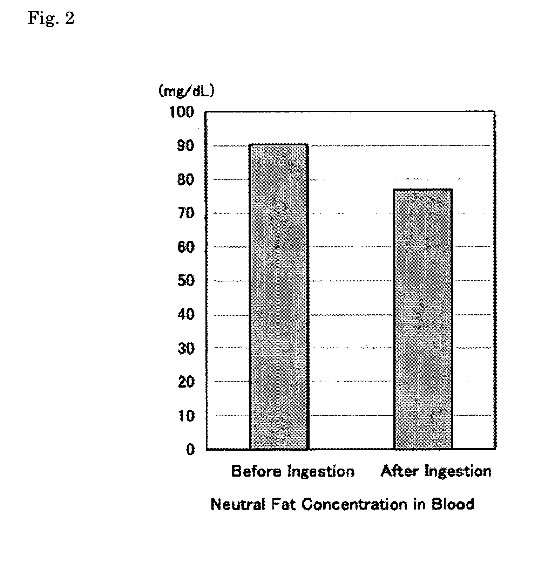

Eighteen subjects consisting of men and women in their twenties to seventies ingested one of the astaxanthin capsules obtained in Preparation Example 3 once a day for four weeks. Blood was collected from each subject before breakfast on the day when the ingestion started, and before breakfast on the day following the day when the ingestion ended, to determine the neutral fat concentration in blood. In the enzymatic method used for this determination, triglyceride (neutral fat) is hydrolyzed by lipoprotein lipase to glycerol. Then, the produced glycerol is further subjected to a colorimetric assay or an UV assay in which an oxidative color-developing agent commonly used in the art is employed in the presence of enzymes such as glycerol kinase and glycerol-3-phosphate oxidase. The results are shown in Figs. 1 and 2. Fig. 1 shows for each subject the change in the concentration of neutral fat in blood before and after the ingestion of the astaxanthin capsules, and Fig. 2 shows the average values of the neutral fat concentration in blood of all the subjects. In both of the graphs, the values on the vertical axis are in mg/dL.

As can be seen from Fig. 1, the concentration of neutral fat in blood was decreased in thirteen subjects (shown by solid lines) out of the eighteen subjects. Moreover, as shown in Fig. 2, the average value of the neutral fat concentrations of all of the subjects was lowered by 14.7% when compared to the average value before the ingestion of the astaxanthin capsules. In particular, in all of the subjects having neutral fat concentrations of 100 mg/dL or more before the ingestion of the astaxanthin capsules, the neutral fat concentrations were lowered (see Fig. 1), and the average drop was 35.2 mg/dL (21.5% when the value before the ingestion was taken as 100%). Thus, it can be seen that a high neutral fat lowering effect was provided especially in the subjects having high neutral fat concentrations. Generally, normal values of the neutral fat concentration are 35 to 149 mg/dL. After the ingestion of the astaxanthin capsules, the neutral fat concentration was reliably lowered in the subjects having high neutral fat concentrations, whereas the neutral fat concentration was not lowered very much in the subjects having low neutral fat concentrations. From these results, it was found that astaxanthin controls the neutral fat concentration to bring this concentration within a normal value range, without lowering excessively the neutral fat concentration, and thus it is also found that astaxanthin works safely for controlling neutral fat concentration in blood. Moreover, the values for neutral fat concentration of almost all of the subjects were within the normal range after the ingestion. This finding showed that astaxanthin controls the concentration of neutral fat in blood so that it is brought within a normal range.

### Reference Example 1: Measurement of 50% Lethal Concentration for HUVEC

Human umbilical vein endothelial cells (HUVECs) (ATCC CRL-1730) were obtained from American Type Culture Collection and precultivated in an Endothelial Cell Growth Medium (CELL APPLICATIONS, USA) containing 10% bovine fetal serum supplemented with 1% Antibiotic-Antimycotic (GIBCO BRL, USA) under a 5% CO₂ atmosphere at 37°C.

A Matrigel matrix (BD Biosciences, USA) was melted and kept at 4°C on ice, and then, 50 µL of the matrix were transferred to each well of a 96-well tissue culture plate. The plate was incubated at 37°C for at least one hour to solidify the matrix solution.

On the other hand, the astaxanthin monoester obtained in Preparation Example 2 was dissolved in dimethylsulfoxide (DMSO), and then diluted with distilled water to prepare stock test solutions in which the astaxanthin monoester was contained in 40 (v/v)% DMSO at 25000, 2500, 250, 25, and 2.5 µM, respectively.

Next, 100 µL of a HUVEC suspension (about 2.5 × 10³ cells/well) were poured into the 96-well Matrigel plate under a 5% CO₂ atmosphere at 37°C. After 24 hours, 100 µL of a growth medium and 2 µL of each of the stock test solutions or the vehicle (40 (v/v)% DMSO) were added to two wells each, and incubated for an additional 72 hours. The final concentrations of the astaxanthin monoester were 250, 25, 2.5, 0.25, and 0.025 µM.

After the incubation, 20 µL of a 90% alamarBlue reagent were added to individual wells, and incubated for an additional 6 hours. Then, the fluorescence intensity of each well was measured at an excitation wavelength of 530 nm and an emission wavelength of 590 nm using a Spectrafluor Plus plate reader to count the number of living cells. This measurement is based on the ability of a living cell to change alamarBlue from the non-fluorescent, oxidized form (blue) to the fluorescent, reduced form (red). The 50% lethal concentration was calculated as the concentration at which the number of living cells was 50% of the number of cells at the start of the experiment.

The result indicates that the 50% lethal concentration (LC₅₀) of the astaxanthin monoester for the HUVECs was 250 µM (maximum concentration of the astaxanthin monoester dissolved in DMSO) or more, and thus it was found that the toxicity of the astaxanthin monoester is low.

According to the present invention, a novel agent for lowering neutral fat concentration in blood and an agent for controlling the concentration of neutral fat in blood are provided. This agent for lowering neutral fat concentration in blood can be used as a therapeutic agent for various diseases having an apparent relation to the level of neutral fat in blood, such as hyperlipemia, arteriosclerosis, hypertension, myocardial infarction, cerebrovascular disorders, cerebral infarction, angina pectoris, pancreatitis, diabetes, obesity, fatty liver, and metabolic disorders. Astaxanthin and/or an ester thereof contained in the agent for lowering neutral fat concentration in blood according to the present invention have been consumed in food for a long time and have low toxicity, therefore, they have a very high degree of safety. Accordingly, the agent for lowering neutral fat concentration in blood according to the present invention is not only used as pharmaceuticals, but also can be used prophylactically on a daily basis as health food products.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. An agent for lowering neutral fat concentration in blood, comprising astaxanthin and/or an ester thereof.

2. A therapeutic agent for a disease having a relation to neutral fat in blood, comprising astaxanthin and/or an ester thereof.

3. The therapeutic agent of claim 2, wherein the disease is arteriosclerosis, a cardiovascular disease, a cerebrovascular disorder, hyperlipemia, or a metabolic disorder.

4. An agent for controlling neutral fat concentration in blood, comprising astaxanthin and/or an ester thereof.

5. Use of astaxanthin and/or an ester thereof in the manufacture of an agent for lowering neutral fat concentration in blood.

6. Use of astaxanthin and/or an ester thereof in the manufacture of a preventive or therapeutic agent for a disease having a relation to neutral fat in blood.

7. The use according to claim 6, wherein the disease is arteriosclerosis, a cardiovascular disease, a cerebrovascular disorder, hyperlipemia, or a metabolic disorder.

8. Use of astaxanthin and/or an ester thereof in the manufacture of an agent for controlling neutral fat concentration in blood.
